Europäisches Patentamt

European Patent Office

Office européen des brevets

(19)

(11) Publication number: **0 315 265**

**A1**

(12)

# EUROPEAN PATENT APPLICATION

(21) Application number: **88202418.5**

(22) Date of filing: **28.10.88**

(51) Int. Cl.⁴: **C07H 13/06**

(30) Priority: **02.11.87 NL 8702614**

(43) Date of publication of application:
**10.05.89 Bulletin 89/19**

(84) Designated Contracting States:
**AT BE CH DE ES FR GB GR IT LI LU NL SE**

(71) Applicant: **COÖPERATIEVE VERENIGING SUIKER UNIE U.A.**
**Zuilenstraat 100**
**NL-4814 ND Breda(NL)**

(72) Inventor: **Van Nispen, Joannes Gerardus Maria**
**Snelliusstraat 22**
**NL-4624 GV Bergen op Zoom(NL)**
Inventor: **Olivier, Aart Pieter Cornelis**
**Berenstraat 17**
**NL-4651 DM Steenbergen(NL)**

(74) Representative: **Baarslag, Aldert D. et al**
**Nederlandsch Octrooibureau**
**Scheveningseweg 82 P.O. Box 29720**
**NL-2502 LS 's-Gravenhage(NL)**

(54) **Method for the preparation of esters of a non-reducing sugar and one or more fatty acids.**

(57) Method for the preparation of esters of a non-reducing sugar and one or more fatty acids in a relatively short time to obtain a sugar product in a high yield and with a desired purity, and, at the same time, restricted investment costs for equipment. Said method is based on the transesterification of at least one fatty acid alkyl ester with a non-reducing sugar in the presence of a transesterification catalyst and, if appropriate, an emulsifier and is characterized by subjecting the starting compounds to a "high shear" mixing treatment at a temperature which is at least equal to the melting temperature of the used fatty acid alkyl ester resulting in a stable homogeneous dispersion, which is then exposed to an elevated temperature in the range of 100-180° C and a reduced presure of at most 0.5 Bar.

EP 0 315 265 A1

## Method for the preparation of esters of a non-reducing sugar and one or more fatty acids

The invention relates to a method for the preparation of esters of a non-reducing sugar and one or more fatty acids by transesterification of one or more fatty acid alkyl esters with a non-reducing sugar in the presence of a transesterification catalyst and, if appropriate, an emulsifier.

Esters of a non-reducing sugar and fatty acids, in particular the mono-esters and di-esters derived from a sugar of this type, are particularly valuable as surface-active agents and possess unique advantages by reason of their composition. For example, surface-active agents of this type are non-toxic, odourless and tasteless, do not irritate the skin and hydrolyse, for example in the human and animal digestive system to give normal nutrition products. In contrast to the majority of the surface-active agents, the esters are based on a sugar of this type and fatty acids are biodegradable under aerobic and anaerobic conditions and, in contrast to the majority of other non-ionic surface-active agents, are solid and therefore easily useable in powder form or in spray-dried products. The esters of a non-reducing sugar and fatty acids are good emulsifying agents and can therefore be used in detergents. Moreover, the esters of a sugar of this type and fatty acids can be used as additives for foodstuffs, cosmetics, pharmaceutical preparations and agricultural products.

It is known that the higher sugar/fatty acid esters, such as the tetra-esters and penta-esters and in particular the hexa-, hepta- and octa-esters, can be used in the treatment of hypercholesterolaemia. Moreover, the hexa-, hepta- and octa-esters can be used as fat replacement agents.

Despite the abovementioned advantages, the esters of a non-reducing sugar and fatty acids have never been used on a large scale because of the problems associated with their preparation. A brief discussion of the methods which had been proposed for the preparation of sugar esters of this type and which, because of technical or economic disadvantages, cannot or can hardly be used on an industrial scale to obtain a product with a competitive cost price relative to the other known surface-active agents is given below.

The most "classical" method for the preparation of esters of a non-reducing sugar and fatty acids comprises the transesterification described, for example, in the Journal of the American Oil Chemist Society, Vol. 34, 1957, pages 185-188, of sucrose with the methyl ester of a fatty acid in a solvent such as dimethylformamide and dimethylsulphoxide, in which both the sugar and the methyl ester of the fatty acid dissolve. This transesterification reaction is carried out in the presence of potassium carbonate as the catalyst and at a temperature of 90°C and under greatly reduced pressure. However, it has been found that the solvents used must, because of the toxicity thereof, be removed as completely as possible, which gives rise to great problems in practice.

To solve the problems associated with the use of the abovementioned solvents for both the sugar and the esters of fatty acids, a method for the preparation of a micro-emulsion system of sucrose and the ester of a fatty acid in propylene glycol is proposed in the Journal of the American Oil Chemist Society, Vol. 44, pages 307-309 (1967). With this method the sugar, in the presence of an emulsifying agent, usually a salt of a fatty acid, and the methyl ester of a fatty acid are dissolved in propylene glycol, after which the solvent is removed under greatly reduced pressure. However, with this method it has been found that it is problematical to bring the reagent into a good micro-emulsion with the desired particle size, while, moreover, the removal of propylene glycol proceeds with difficulty. At the same time, propylene glycol esters are obtained as a by-product with this method.

A later modification of the solvent-transesterification process, with which water is used as the solvent is described in British Patent Specification 1,332,190. With this method the sugar is completely dissolved in the water in the presence of a fatty acid soap, a fatty acid ester and a transesterification catalyst, after which the mixture id dehydrated under reduced pressure and at elevated temperature so that a homogeneous melt is obtained. This method also presents problems in respect of the warming of the water-containing product under reduced pressure, during which operation the pressure must be regulated carefully as a function of the temperature in order to prevent hydrolysis of the fatty acid ester. For these reasons, this method is undesirably complicated for use on an industrial scale.

A solvent-free transesterification method is also described in J. Amer. Oil. Chem. Soc. 1970, 47, pages 56-60. With this method sucrose is used in the molten state, so that the method is carried out at a temperature of 170-190°C. After a short time, however, the sugar starts to degrade to a black, tar-like mass, so that the reaction with the fatty acid alkyl ester necessarily has to take place very rapidly. Usually the reaction is complete within 20 minutes, and sometimes even after only 2 minutes. The reaction must be carried out in the presence of an alkali metal-free and anhydrous soap, which serves for solubilization of the fatty acid alkyl ester in the molten sugar and for catalysis of the transesterification. Alkoxides, alkali and ordinary soaps are completely unsuitable as the catalyst as the presence thereof results in a very rapid decomposition of the sugar and gives rise to a black coloration of the mixture. In view of the problems associated with the control of the reaction, as the reaction has to be completed very rapidly in order to prevent degradation of the sugar, this reaction can only be carried out on a laboratory scale and offers little prospect for use on an industrial scale.

A method for the preparation of a surface-active agent by the reaction of solid, particulate sucrose with at least one triglyceride in the presence of a basic transesterification catalyst at a temperature of 110-140°C under atmospheric pressure and in the absence of any solvent is known from British Patent Specification 1,399,053. As little water as possible must be present in the starting materials, since about 1% by weight water already greatly retards the course of the reaction due to the formation of lumps of sugar and, moreover, gives rise to an acceleration of the soap formation. According to a preferred embodiment, in the method described in British Patent Specification 1,399,053 both the initiation period and the reaction period are considerably shortened by the addition of an emulsifying agent to the reaction mixture, advantageously in an amount of 5-10% by weight. In addition to di- and mono-glycerides, the crude end product of this method, which contains surface-active substance, can also be used. However, it has been found that the reaction proceeds very slowly and takes about 8 hours or more.

In addition, a method for the preparation of surface-active substances containing sugar esters is described in British Patent Application 2,065,634, with which method solid particulate sucrose, at least one triglyceride of a fatty acid having at least 8 carbon atoms and a basic transesterification catalyst are reacted at a temperature of 110-140°C under atmospheric pressure. However, the starting mixture must contain at least 10% by weight fatty acid soap, while the optimum soap concentration in the starting material is 25-30% by weight. Moreover, this soap must consist to at least 50% of potassium soap. This method therefore has disadvantages, for example in that the end product is contaminated to a considerable extent with soap, in particular potassium soap.

A method for the preparation of sucrose fatty acid esters is known from British Patent Application 2,161,806 with which, according to the embodiments given in the examples and also to the method given in the description of this British Patent Application as a preferred embodiment, the reactants sucrose and the fatty acid alkyl ester are added to a molten fatty acid soap, such as sodium stearate, at a temperature of at least 70°C and preferably 90-110°C. The actual transesterification then takes place at a temperature of 120-180°C under a reduced pressure of at most 10 mmHg while stirring with a linear speed of 1.0-50 m/s. This high linear speed of the stirring device serves only, as can be seen from the context of this British Patent Application 2,161,806, to prevent phase separation and the occurrence of foaming of the reaction mixture under the abovementioned reaction conditions. As examples of types of stirrers, a propeller stirrer and a turbine stirrer are used in the examples, which represent an embodiment of the invention disclosed in this British Patent Application. The method according to this British Patent Application has, however, in the first place the disadvantage that a fairly high content of emulsifier has to be used. When an alkali metal fatty acid soap is used as the emulsifier, the amount of emulsifier to be used is 3-15% by weight and preferably even 5-10% by weight, calculated on the total weight of the sucrose and the fatty acid alkyl ester, and in the case of sucrose fatty acid esters 3-30% by weight and preferably 5-15% by weight, calculated on the total weight of the sucrose and fatty acid alkyl ester used as starting material, is employed. The imperative molar ratio of sucrose: fatty acid alkyl ester of at least 1:4, so that sucrose fatty acid esters with a degree of substitution of, for example, 1 (monoesters) are virtually excluded, is mentioned as a second disadvantage; as sucrose possesses eight hydroxyl groups per molecule, the degree of substitution can vary from 1 to 8. Moreover, the relatively large amount of fatty acid alkyl ester which in the first stage, for the preparation of a melt with a temperature of at least 70°C and preferably 90-110°C, is present in liquid form, will make up a substantial portion of the melt which is subjected to the actual transesterification reaction. Finally, the method according to this British Patent Application 2,161,806 is carried out batch-wise, which is a disadvantage for implementation on an industrial scale in view of the high investment costs for equipment which must be made per unit end product.

Finally, a method for the preparation of esters of a non-reducing sugar and at least one fatty acid is known from the Applicant's European Patent 190,779, with which the sugar, the fatty acid alkyl ester and a transesterification catalyst and also a substantial amount of emulsifier, such as potassium stearate or sodium stearate, are fed through a worm shaft reactor, which is known per se, operating at elevated temperature and pressure, after which the mass which is obtained from the worm shaft reactor and which is virtually completely molten is further reacted in a separate vessel under highly reduced pressure and at elevated temperature. However, in view of the considerable amount of emulsifier which, as can be seen from the example given in the abovementioned European Patent, is 10% by weight, this component can present problems when working up the end product and in some cases can result in a laborious working-up. In addition, this last discussed method has the disadvantage that the process sequence clearly takes place in two separate steps, i.e. firstly in a worm shaft reactor at elevated temperature and pressure and then in a vessel at elevated temperature and reduced pressure. This means that this known method cannot or can hardly be carried out continuously, which must be regarded as disadvantageous for an industrial production procedure. Moreover, this known method requires the use of a worm shaft reactor, which necessitates relevant investment costs for equipment.

To summarize, it can be stated that the solutions proposed in the state of the art discussed above with regard to the problems relating to the preparation of esters of a non-reducing sugar on the one hand and one or more fatty acids on the other hand offer insufficient consolation.

A method has therefore been sought with which a non-reducing sugar and fatty acid alkyl esters can be reacted with one another in a short time to obtain a sugar ester product in a high yield and with a desired purity, and, at the same time, the investment costs for equipment must remain restricted and the method can be carried out continuously with a view to an industrial production procedure.

Surprisingly, it has been found that the aim described above can be achieved when the starting mixture of the reactants is subjected to a "high shear" mixing treatment at a temperature which is at least equal to the melting temperature of the fatty acid alkyl ester used as starting material, and the homogenous dispersion thus obtained is then exposed to an elevated temperature in the range of 100-180°C and a reduced pressure of at most 0.5 bar. Since the temperature during the "high shear" mixing treatment according to the invention is at least equal to the melting temperature of the fatty acid alkyl ester used as starting material, the non-reducing sugar and the transesterification catalyst and also any emulsifier which may be present are dispersed in the liquid fatty acid ester phase during this treatment, which phase, contrary to expectation, remains stable in the second step of the method according to the invention, that is to say no or virtually no phase separation occurs, which is essential for a favourable course of the transesterification reaction between the fatty acid alkyl esters and the sugar.

The abovementioned term "high shear mixing treatment" is understood to mean the action of a "high shear" mixing device. Devices of this type, which are available commerically, possess a high dispersion power. The principle of these devices is based on the effects of the mechanical high frequency on stator/rotor mixing head systems with high peripheral speeds of the rotor, for example in the order of magnitude of 15-20 m/s and above. As a result of the compression and decompression waves generated in these systems, a controlled cavitation is generated in the system. More particularly, the action of a "mixing head" based on a stator/rotor system can be represented as follows:

1) The constituents to be mixed with one another are subjected, in the mixing head, to an intense hydraulic shear caused by the high speed of rotation of the rotor within the restricted space of the stator.

2) The centrifugal force generated by the rotor sucks the solid and liquid constituents towards the mixing head where they are ground in the free space, accurately made to measure, between the ends of the rotor blades and the inner wall of the stator.

3) Further centrifugal force drives the constituents out of the mixing head, while they are subjected to a mechanical shear between the ends of the rotor and the sharp edges of the openings in the stator.

4) Finally, as a result of the same centrifugal force, the contents of the mixing head are propelled via the outlet of the device into a discharge line while, at the same time, new constituents are drawn in via the feed line to keep the mixing head continuously fed.

As mentioned, the stator has openings, which can have many shapes, such as circular, rectangular, etc.

The "high shear" mixing devices referred to above are available commerically and are marketed, for example, by the manufacturers Silverson Machines Ltd., Chesham, England, as Silverson devices, Janke en Kunkel GmbH u. Co., Staufen, West Germany, Ultra-Turrax devices, and by Supraton F.J. Zucker GmbH, Neuss, West Germany, as Supraton dispersion devices.

As is known, the "high shear" mixing devices differ from the conventional stirring and mixing devices in their action. In the article "Dispergieren im Gradienten-Impulsverfahren" ("Dispersing in Gradient Pulse Processes") by Wiedemann and Blencke, Chem. Anlagen und Verfahren, No. 4, 1976, pages 82-86, 89, 90 and 110 the specific energy supply ($\Sigma$), which is expressed in kJ/kg, is defined as the characteristic property. This specific energy supply $\Sigma$ is considerably higher for the "high shear" mixing devices than for conventional stirrer devices. More particularly, this unit $\Sigma$, which

is the quotient of the power P of the device and the displacement capacity Q of the stirrer, i.e. $\Sigma = P/Q$, is in the range 1-10, in particular 2-7, for "high shear" stirring devices.

For comparison, the specific energy supply $\Sigma$ for a conventional turbine stirrer is calculated below. This calculation is based on standard geometry, i.e.
- the height/vessel diameter ratio = 1
- the stirrer diameter/vessel diameter ratio = 1/3
- the vessel capacity = 1 m³
- the stirrer diameter (d) = 0.36 m
- the power of the stirrer device (P) = 10 kW
- the density of the mass to be stirred ($\rho$) = 800 kg/m³

The value of the speed of revolution is required to calculate the displacement capacity Q:
(speed of revolution = $(P / \rho \cdot (d)^5 \cdot Np)^{1/3}$
where Np represents the power characteristic of the stirrer element (turbine stirrer) with a value 6; the speed of revolution is 7 revolutions/sec.

The specific energy supply $\Sigma = P/Q$
$\Sigma = P / \text{pump capacity} \cdot \rho$
$\Sigma = P / (1.3 \times \text{speed of revolution} \times d^3) \cdot \rho$
$\Sigma = 0.03$ kJ/kg

As can be derived from the above comparison, for a conventional turbine stirrer with a power of 100 kW, which is improbably high for a 1 m³ vessel, a value of only 0.3 is reached for $\Sigma$, so that the abovementioned term specific energy supply is a suitable unit for distinguishing between the conventional stirring devices on the one hand and the "high shear" mixing devices on the other.

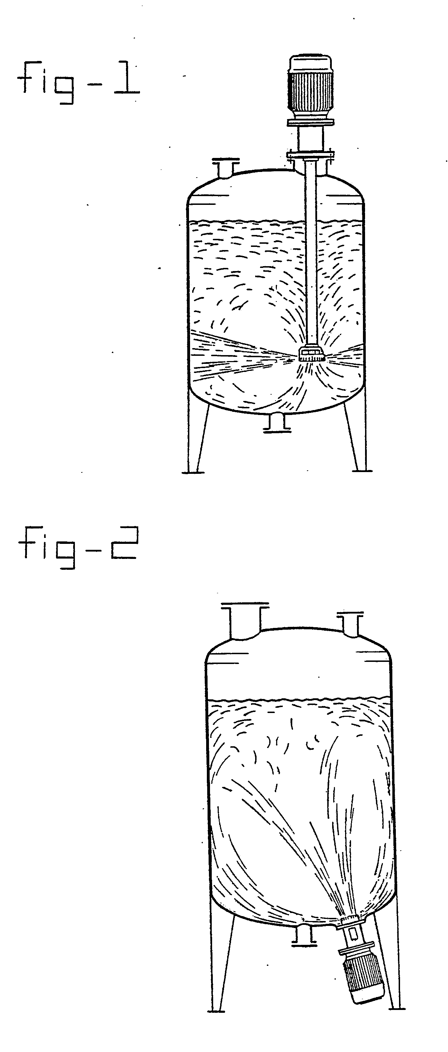

For example, the "high shear" mixing device is in a pressure vessel, in which a vacuum can also be generated. In this way, the "high shear" mixing treatment and the subsequent step of the method according to the invention, which is carried out at elevated temperature and greatly reduced pressure, can be carried out in a single vessel, if desired while maintaining the "high shear" mixing treatment. An embodiment of this type is shown in Figures 1 and 2.

The advantages of the method according to the invention over the methods known from the prior art are:

1) simple to carry out from the technological standpoint;

2) the method can be carried out fully continuously;

3) the method requires a short reaction time;

4) the occurrence of side reactions is greatly reduced; and

5) working up of the reaction product is, if considered necessary, simple. More particularly, a working up of this type can be superfluous because of the low emulsifier content and depends on the standards laid down in the legislation relevant to the use of products of this type.

A significant advantage of the method according to the invention lies in the possibility that this can be carried out continuously, so that the investment costs for equipment can be kept relatively low and, therefore, an end product with a relatively attractive cost price can be obtained. This aspect of an attractive cost price is further emphasized by the surprisingly low amount of emulsifier required with the method according to the invention, which amount, for example, can be less than 3% by weight, calculated on the total weight of the sugar and the fatty acid alkyl ester. A continuous procedure of the method according to the invention can, for example, be carried out in three steps, i.e.

(a) the "high shear" mixing treatment in a vessel provided with a "high shear" mixing device;

(b) the actual transesterification reaction of the stable homogeneous dispersion obtained under step (a) under substantially reduced pressure and at elevated temperature in a second vessel; in this vessel the dispersion is stirred with a conventional stirrer until the viscosity of the reaction mass has reached a certain value; and

(c) the continuation of the transesterification reaction of the viscous reaction mass obtained in step (b) in a third vessel which is fitted with a stirrer suitable for stirring viscous masses.

With the aid of a continuous embodiment of this type of the method according to the invention it is therefore possible to feed the starting components to step (a) and to remove the sugar esters from step (c).

With regard to the emulsifier which may be employed in the method according to the invention it should be noted that this amount may not be so large that the reaction mass becomes too viscous. The content of, for example, alkali metal soap, such as potassium stearate, in the reaction mass must therefore normally be at most 20% by weight, for example 0-5% by weight and advantageously only 0-3% by weight, calculated on the total weight of the sugar and the fatty acid alkyl ester. Examples of emulsifiers which can preferably be used with the method according to the invention are potassium stearate and sodium stearate. However, many other suitable types of emulsifiers can also be used.

As has been mentioned above, the temperature of the reaction mass used in the "high shear" mixing device must be at least equal to the melting temperature of the fatty acid alkyl ester used as starting component. In this way, a liquid phase is obtained in which the non-reducing sugar and the transesterification catalyst are dispersed in a surprisingly stable manner.

Transesterification catalysts which can be used are, for example, potassium hydroxide, sodium hydroxide and lithium hydroxide, potassium carbonate, sodium carbonate and lithium carbonate, as well as the potassium, sodium and lithium derivatives of bicarbonate, methanolate, ethanolate and propanolate. Advantageously, potassium hydroxide and in particular potassium carbonate are used. The amount of transesterification catalyst to be used in the starting mixture is usually 0.1-10% by weight, calculated on the total weight of the sugar.

The method according to the invention is preferably carried out with a methyl ester of a fatty acid as one of the starting materials. In this way, esters of sugar and fatty acid can be obtained in very pure form. Another advantage is that the alcohol formed during the reaction, i.e. methanol, is the most advantageous alcohol with regard to the removability from the reaction mixture. As a result of removal of the alcohol, the equilibrium reaction shifts towards the sugar ester side. However, in addition to the methyl esters, it is also possible to use ethyl esters and propyl esters of fatty acids.

The fatty acid alkyl esters preferably used as starting material in the method according to the invention normally contain 8-22 carbon atoms in the fatty acid moiety. Furthermore, the fatty acids can be straight-chain or branched and saturated or unsaturated. Examples of fatty acids of this type are palmitic acid, stearic acid, oleic acid etc. In addition, mixed fatty acid alkyl esters or fats can be used.

Any commercially available solid sugar, such as sucrose and sorbitol, of any quality and size can be used as the non-reducing sugar. Preferably, however, grains with a size of 0.1 mm or less are used.

After the action of the "high shear" mixing device on the reaction mass, the stable, homogeneous dispersion obtained is subjected to the actual transesterification reaction. The temperature employed in this reaction is in the range 100-180°C and is preferably 140-160°C. The pressure employed in this step is not more than 0.5 bar, but is preferably in the range 0-0.2 bar, in particular 0-0.1 bar.

The sugar esters obtained with the method according to the invention are normally a mixture of mono-esters and higher esters. For example, sucrose possesses eight hydroxyl groups per molecule, so that the number of fatty acid groups which can be bonded to a sucrose molecule can vary from 1 to 8. The composition of a sugar-ester mixture prepared is therefore partly dependent on the mutual ratio of the sugar used as starting component and the fatty acid alkyl ester(s) used as starting material, which can vary over a wide range. For example, the weight ratio of sucrose to fatty acid alkyl ester(s) can vary from 80:20 to 5:95.

The invention is illustrated in more detail with the aid of the exemplary embodiments given below, which, however, must not be regarded as restrictive.

## Example I

A mixture of 475 g sucrose with a mean particle size of 0.1 mm, 475 g methyl stearate, 25 g potassium carbonate and 25 g potassium stearate was subjected to the action of a "high shear" mixing device of the Silverson type at a temperature of 50°C. The speed of rotation of the rotor was 10 m/s and the stator or disintegrater head was provided with circular openings with a cross-section of 10 mm. Directly after this treatment, which took 10 minutes, the homogeneous dispersion obtained was exposed to a temperature of 140°C and a pressure of 0.01 bar for 180 minutes, while mixing. Under these conditions, the yield of the sugar esters was 65% by weight, calculated on the starting components.

The specific energy supply $\Sigma$ of the Silverson device with a power of 0.4 kW and a displacement capacity of 0.25 kg/s had a value of 1.6 kJ/kg.

## Example II

A mixture of 610 g sucrose (with a mean particle size of 0.1 mm), 340 g methyl stearate, 25 g potassium stearate and 25 g potassium carbonate was reacted under the conditions given in Example I. The amount of sugar esters formed was 53% by weight, calculated on the starting components.

## Example III

A mixture of 5 kg sucrose with a mean particle size of 0.1 mm, 5 kg methyl stearate, 0.26 kg potassium carbonate and 0.26 kg sugar esters containing 17% by weight of mono-esters was subjected to the action of the type Dispax DR 3-6/6-P at a temperature of 120°C. The Dispax is a "high shear mixing device" with a throughput of at most 4 m³/h, based on water. The speed of rotation of the rotor shaft was 8000 min⁻1, which signifies a maximum rotor circumferential speed of about 25 m/s. The dispersion space consisted of 3 rotor/stator spaces in series.

The mixture of starting materials was circulated for 10 minutes with the aid of the Dispax over a reaction vessel provided with an anchor stirrer as the mixing device. Directly after the high-shear treatment, the homogeneous dispersion obtained was exposed to a temperature of 150° C and a pressure of 50 mbar for 300 minutes in the reaction vessel, while stirring.

Under these conditions the yield of sugar esters was 57% by weight, calculated on the starting components. Calculated on the total amount of sugar esters, 6.0% by weight mono-esters, 17.5% by weight di-esters, 32.5% by weight tri-esters and 44.0% by weight higher esters were found. The specific energy supply Σ of the Dispax device with a power of 3 kW and a displacement capacity of 0.56 kg/s had a value of 5.4 kJ/kg.

3% by weight stearate, calculated on the starting components, was detected in the end product.

Example IV

A mixture of 6 kg sucrose with a mean particle size of 0.1 mm, 3 kg methyl stearate, 0.75 kg potassium carbonate and 0.25 kg potassium stearate was reacted under the conditions given in Example III. After a reaction time of 3 hours, the amount of sugar esters formed was 27.5% by weight, calculated on the starting components. Calculated on the total amount of sugar esters, 60.0% by weight mono-esters, 31.0% by weight di-esters and 9.0% by weight tri-esters were found.

Example V

A mixture of 3.2 kg sucrose with a mean particle size of 0.1 mm, 6.3 kg methyl stearate, 0.25 kg potassium carbonate and 0.25 kg potassium stearate was reacted under the conditions given in Example III. After a reaction time of 5 hours, the amount of sugar esters formed was 76.0% by weight, calculated on the starting components. Calculated on the total amount of sugar esters, 6.5% by weight mono-esters, 18.5% by weight di-esters, 35.0% by weight tri-esters and 40.0% by weight higher esters were found.

Example VI

A mixture of 3.2 kg sucrose with a mean particle size of 0.1 mm, 6.3 kg methyl stearate, 0.25 kg potassium carbonate and 0.25 kg sugar esters containing 17% by weight mono-esters was reacted under the conditions given in Example III. After a reaction time of 5 hours the amount of sugar esters formed was 58.5% by weight, calculated on the starting components. Calculated on the total amount of sugar esters, 3.5% by weight mono-esters, 14.5% by weight di-esters and 30.0% by weight tri-esters and 52% by weight higher esters were found. 3.6% by weight stearate, calculated on the starting components, were detected in the end product.

**Claims**

1. Method for the preparation of esters of a non-reducing sugar and one or more fatty acids by transesterification of at least one fatty acid alkyl ester with a non-reducing sugar in the presence of a transesterification catalyst and, if appropriate, an emulsifier, characterized in that the mixture of the starting components is subjected to a "high shear" mixing treatment at a temperature which is at least equal to the melting temperature of the fatty acid alkyl ester used as starting material, and the homogeneous dispersion thus obtained is then exposed to an elevated temperature in the range of 100-180° C and a reduced pressure of at most 0.5 bar.

2. Method according to Claim 1, characterized in that sucrose or sorbitol is used as the non-reducing sugar.

3. Method according to Claim 1 or 2, characterized in that fatty acid alkyl esters which are derived from a fatty acid with 8-22 carbon atoms and an alcohol with 1, 2 or 3 carbon atoms are used as the starting material.

4. Method according to Claim 3, characterized in that a methyl ester of a fatty acid with 8-22 carbon atoms is used as the fatty acid alkyl ester.

5. Method according to one or more of Claims 1-4, characterized in that the dispersion obtained is exposed to a temperature in the range of 140-160° C and a pressure of 0-0.2 bar.

6. Method according to Claim 5, characterized in that the dispersion obtained is exposed to a temperature in the range of 140-150° C and a pressure of 0-0.1 bar.

7. Method according to one or more of Claims 1-6, characterized in that the method is carried out in a single vessel.

8. Method according to one or more of Claims 1-7, characterized in that the method is carried out continuously.

9. Method according to one or more of Claims 1-8, characterized in that an alkali metal salt of a fatty acid having 8-22 carbon atoms is used as the emulsifier.

10. Method according to Claim 9, characterized in that potassium stearate or sodium stearate is used in an amount of 0-5% by weight, calculated on the total weight of the sugar and the fatty acid alkyl ester.

11. Method according to Claim 10, characterized in that potassium stearate or sodium stearate is used in an amount of 0-3% by weight, based on the total weight of the sugar and the fatty acid alkyl ester.

fig-1

fig-2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| Y | US-A-3 963 699 (G.P. RIZZI) * Column 6, line 25 - column 7, line 9; claims 1-13 * --- | 1-11 | C 07 H 13/06 |
| Y,D | EP-A-0 190 779 (COOPERATIEVE VERENIGING SUIKER) * Column 3, line 10 - column 5, line 55; claims 1-7 * --- | 1-11 | |
| A,D | GB-A-2 161 806 (DAI-ICHI) * Whole document * --- | 1 | |
| A,D | JOURNAL OF THE AMERICAN CHEMICAL SOCIETY, vol. 47, no. 2, 1970, pages 56-60; R.O. FEUGE et al.: "Preparation of sucrose esters by interesterification" * Whole article * ----- | 1 | |

TECHNICAL FIELDS
SEARCHED (Int. Cl.4)

C 07 H 13/00

The present search report has been drawn up for all claims

| Place of search | Date of completion of the search | Examiner |
|---|---|---|
| THE HAGUE | 19-01-1989 | BRENNAN J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)